(19) **Europäisches Patentamt**

**European Patent Office**

**Office européen des brevets**

(11) **EP 1 343 014 A2**

(12) **EUROPEAN PATENT APPLICATION**

(43) Date of publication:
**10.09.2003 Bulletin 2003/37**

(51) Int Cl.[7]: **G01N 33/88**, G01N 33/68

(21) Application number: **03251364.0**

(22) Date of filing: **06.03.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **06.03.2002 US 91813**

(71) Applicant: **JOHNSON & JOHNSON CONSUMER COMPANIES, INC.**
**Skillman, NJ 08558 (US)**

(72) Inventors:
- **Huang, Kelly**
  **Hillsborough, NJ 08844 (US)**
- **Tierney, Neena**
  **Yardley, PA 19067 (US)**
- **Wiegand, Benjamin**
  **Newtown, PA 18940 (US)**

(74) Representative: **Mercer, Christopher Paul et al**
**Carpmaels & Ransford**
**43, Bloomsbury Square**
**London WC1A 2RA (GB)**

(54) **Method and kit for measuring skin inflammation or irritation**

(57) A non-invasive, *in vivo* method for measuring sub-clinical or clinical inflammation or irritation of mammalian skin from exposure of the skin to a topical skin care product, exposure to an external aggression or combinations thereof is disclosed. In one embodiment, the method includes the steps of collecting eicosanoid from the skin using a non-invasive collection device and analyzing levels of eicosanoid collected from the skin. A kit for measuring a marker of skin irritation or inflammation is also disclosed. The kit includes a non-invasive collection device for collecting secretions from the skin surface, and an immunoassay for measuring level of eicosanoid in the secretions.

**EP 1 343 014 A2**

**Description**

FIELD OF THE INVENTION

This invention relates to a method and kit for measuring skin inflammation or irritation. More particularly, the invention relates to a non-invasive, *in vivo* method and kit for measuring skin inflammation or irritation.

BACKGROUND OF THE INVENTION

Skin is the largest organ in the body, and its appearance has a large effect on one's self-confidence and quality of life. In fact, an initial impression of someone, which is long lasting, is often driven by the visual appearance (facial features, hair color, etc.) of the individual.

Consumers use skin care products to treat their skin conditions (*e.g.,* acne, psoriasis, fine lines and wrinkles), improve skin health (*e.g.*, dry, flaky skin; oily skin) or cover skin blemishes. There has been a tremendous amount of continued research into the development of new actives to improve the speed and efficacy of treatment of these various skin conditions, which has led to a plethora of new skin care formulations. These products often deliver against a specific skin care end benefit but need a longer time frame (eight to twelve weeks or longer) to maximize this benefit.

As the end benefit of these topical skin care products is being delivered over an extended period of time, it would be desirable to develop measurement capabilities to determine the irritation profiles of these products without having to test them with consumers over the extended time period. In fact, sensitive measurements that could determine the irritation profile after a single use, when there are no visual by-products (*i.e*., sub-clinical effects), such as inflammation, irritation, oedema, and erythema, would be highly preferred. In addition, exposure to external aggressions such as abrasion, friction, scrubbing, sun exposure, wind exposure, smoke exposure, other environmental exposures or the application and removal of an adhesive device such as a bandage to and from the skin, can also cause skin damage leading to inflammation or irritation, the degree of which is susceptible to measurement. The present invention provides a novel method and apparatus for measuring and quantifying such sub-clinical or clinical effects.

The inflammatory response of the skin caused by exposure to a skin care product or exposure to an external aggression is modulated by the keratinocytes within the skin primarily via two distinct pathways: the cytokine and the arachidonic acid pathway. Within these pathways, the various mediators that are expressed, secreted, or released are considered to be either a primary or secondary inflammatory mediator. Interleukin-1 alpha (IL-1$\alpha$) is considered to be a primary pro-inflammatory mediator within the cytokine pathway, whereas prostaglandin $E_2$ ($PGE_2$) is considered to be a primary pro-inflammatory mediator of the arachidonic acid pathway.

There are two main approaches for models that exist for the measurement of irritation or inflammation, *i.e., in vitro* or *in vivo* measures. A review article entitled "Strategies for the assessment of acute skin irritation potential" (Robinson *et al.,* in *J. Pharmacol. Toxicol.,* 1999) provides a good overview of current *in vitro* skin corrosion and irritation testing methodologies, and is incorporated herein in its entirety by reference. A representative skin corrosion and irritation testing methodology is disclosed in US-A-6,020,148, the disclosure of which is incorporated herein in its entirety by reference, which discloses the use of an *in vitro* method to measure the ocular or dermal irritation of topical skin care products. The ocular or dermal irritation is quantified by measuring the viability of a model cell culture system after application of the skin care product. Different end markers can be measured using this technique, which can be monitored over time. Another methodology is disclosed in EP-A-0,497,39, the disclosure of which is incorporated herein in its entirety by reference, which discloses the use of an *in vitro* model comprising a human skin co-culture of keratinocytes and fibroblasts to evaluate the irritancy of surfactants via cytotoxicity and pro-inflammatory endpoints. While these methods provide directional information on potential irritation or other clinical safety issues, the *in vitro* models do not accurately represent inflammation and irritation exhibited by human skin.

In addition, *in vivo* approaches have also been used to assess clinical safety of skin care products. Examples of *in vivo* approaches include: 4 hour patch testing (See Robinson, *et al.* review or Robinson *et al.,* in *Contact Dermatitis,* 1998, "Application of a 4-h human patch test method for comparative and investigative assessment of skin irritation") with the assessment of erythema after product patching; the use of suction blister fluid to measure IL-1$\alpha$ and eicosanoids after 24 hour patch exposure (Muller-Decker *et al., in Toxicology and Applied Pharmacology,* 1998, "Arachidonic Acid Metabolism in Primary Irritant Dermatitis Produced by Patch Testing of Human Skin with Surfactants"); and the use of tape stripping and capsaicin to measure eicosanoid and cytokine levels in acute skin irritation (Reilly and Green, in *Acta. Derm. Venereol.*, 1999, "Eicosanoid and Cytokine Levels in Acute Skin Irritation in Response to Tape Stripping and Capsaicin"). An additional approach taught by Rheins, *et al.* WO 00/10579, "Method for Detection of Biological Factors in Epidermis" teaches the user to scrape the skin or use adhesive tape to strip the skin and then analyze for specific polynucleotides/cytokines. While these methods use human models, they involve an exaggerated use condition or a high degree of invasiveness during the procedure, which limits their usefulness as a clinical assessment tool. This is especially relevant, when evaluating sub-clinical irritation.

Over the last several years, Robinson *et al.* have disclosed non-invasive methodologies using Sebutape® (Cuderm Corporation, Dallas, Texas), an adhesive-coated microporous plastic film, to absorb pro-inflammatory cytokines as a marker of inflammation. In 1997, it was demonstrated that this methodology could be applied to the measurements of irritation on different body sites, as well as for individuals of different ages (Perkins, *et al.,* Meeting of the Society of Toxicology, 1997, "Development of a noninvasive method for assessing human skin irritation"). Subsequent development showed that these inflammatory markers may correlate with skin conditions. Examples of the application of this methodology include the following: the correlation of Interleukin-1 receptor antagonist (IL-1ra) and diaper rash severity (Perkins, *et al.,* Society of Toxicology Annual Meeting, 1998, "Further Development of a noninvasive method for assessing human skin irritation"), the correlation of IL-1$\alpha$ and sub-clinical irritation (without visible erythema) due to sodium lauryl sulfate (SLS) application (Perkins, *et al.*, Society of Investigative Dermatology Annual Meeting, 1999, "Noninvasive method for assessing inflammatory changes in chemically treated human skin"), and the assessment of the severity of inflammatory scalp conditions (Cardin, *et al.,* Society of Toxicology Annual Meeting, 2001, "Development of a noninvasive method for recovery of molecular markers of normal and compromised scalp conditions"). One of the potential issues that could arise when using interleukins or other proteins as a marker of inflammation is their potential interaction with the skin care product being tested. For example, it is thought that the interactions of these proteins with anionic surfactants found in some skin care products can lead to protein denaturation, which could affect their apparent levels during analysis. Therefore, the development of a lipid-based marker that would retain stability in the presence of a wide variety of skin care products or other test compounds would be preferred and would overcome the above-mentioned difficulties associated with using proteins, such as interleukins, as an inflammation/irritation marker. In addition, the method should be able to measure not only the effects of topical skin care products on the skin, but also the effects of exposure to the above-referenced external aggressions.

BRIEF SUMMARY OF THE INVENTION

In one embodiment, the present invention is directed to a method for measuring a marker of sub-clinical or clinical inflammation or irritation of mammalian (animal or human) skin by the collection and measurement of the amount of an eicosanoid, preferably, prostaglandin, more preferably, prostaglandin $E_2$ ($PGE_2$), present on the skin surface. The method of the invention, including the steps of:

(a) collecting secretions from the surface of the skin using a non-invasive collection procedure, said non-invasive collection procedure utilizing a non-invasive collection device; and
(b) analyzing the level of at least one eicosanoid in the secretions collected from the skin surface by said device.

The measurement of eicosanoids provides the needed sensitivity to differentiate between very mild skin care products or exposure to external aggressions, while obviating any concern of protein denaturation associated with prior art methods.

Another embodiment of the invention is directed to a method of measuring the sub-clinical or clinical inflammation or irritation of mammalian skin due to exposure of the skin to a topical skin care product, an external aggression or combinations thereof. This embodiment of the method of the invention, includes the steps of:

(a) collecting secretions from the surface of said skin using a non-invasive collection procedure including a non-invasive collection device;
(b) measuring a baseline level of eicosanoid in the secretions collected from the surface of said skin;
(c) exposing said skin to at least one topical skin care product, to at least one external aggression or combinations thereof;
(d) collecting secretions from the surface of said skin using a non-invasive collection procedure including a non-invasive collection device after step (c);
(e) measuring the level of eicosanoid in the secretions collected from the surface of the skin after step (c); and
(f) comparing the level of eicosanoid determined in step (e) with the level of eicosanoid determined in step (b).

Preferably, the eicosanoid is prostaglandin and, more preferably, is prostaglandin $E_2$. Preferably, step (d) of the method is performed about 24 hours after step (c).

The non-invasive collection device may include a device selected from the group consisting of an uncoated non-porous plastic film, an uncoated microporous plastic film, an adhesive-coated non-porous plastic film, an adhesive-coated microporous plastic film, a woven fibrous web, a non-woven fibrous web, a natural sponge, a synthetic sponge and a plastic foam.

In another embodiment, the method further includes the step of analyzing the level of at least one cytokine in the secretions collected from the skin surface by the device. Preferably, the cytokine is interleukin-1$\alpha$. Most preferably,

the cytokine is interleukin-1$\alpha$ and the eicosanoid is prostaglandin E$_2$.

In yet another embodiment, the method further includes the step of measuring the level of protein in the skin secretions and normalizing the level of eicosanoid to the level of protein.

Another aspect of the invention is directed to a kit for measuring markers for clinical or sub-clinical inflammation or irritation of mammalian skin. The kit includes:

(a) a non-invasive collection device for collecting secretions from the surface of the skin; and
(b) an immunoassay for measuring levels of eicosanoid in the secretions.

BRIEF DESCRIPTION OF THE DRAWINGS

Fig. 1 is a calibration curve for an immunoassay for the determination of PGE$_2$ used in some embodiments of the invention.

Fig. 2 is a calibration curve for an immunoassay for the determination of IL-1$\alpha$ used in some embodiments of the invention.

DETAILED DESCRIPTION OF THE INVENTION

The levels of a specific marker, such as a cytokine (including interleukin-1$\alpha$) or an eicosanoid (including a prostaglandin, exemplified by PGE$_2$) expressed or secreted on the skin, may be used to quantify sub-clinical or clinical skin irritation or inflammation induced on the skin by a topical skin care product, by exposure of the skin to an external aggression or combinations thereof. In addition, measuring a combination of markers, such as an eicosanoid and a cytokine, may be used to provide a more complete picture of the amount of sub-clinical or clinical skin irritation or inflammation.

As used herein, the term "topical skin care product" refers to a personal cosmetic, toiletry, or healthcare product such as dry or wet wipes, washes, baths, shampoos, gels, soaps, sticks, balms, sachets, pillows, mousses, sprays, lotions, creams, cleansing compositions, powders, oils, deodorants, bath oils and other bath compositions which may be added to a bath. Topical skin care products may also include, but are not limited to, aerosols, candles, and substances that may be used with vaporizers. The aforementioned topical skin care products are well known to those skilled in the art.

As used herein, the term "external aggression" refers to a stimulus other than a personal care product that can cause irritation or inflammation to the skin. Illustrative examples of external aggressions include abrasion, friction, scrubbing, sun exposure, wind exposure, smoke exposure, other environmental exposures or the application and affixing and removal of an adhesive-coated device such as an adhesive bandage to and from the skin.

As used herein, the term "non-invasive collection procedure" refers to a procedure for collection of secretions from the skin surface that does not cause visible erythema during collection.

As used herein, the term "non-invasive collection device" refers to a device for collection of secretions from the skin surface that does not cause visible erythema during collection.

The method of the invention measures the irritation of the skin in response to reatment with a topical skin care product, exposure to an external aggression or a combination thereof. The topical skin care product may be applied to the skin with a wide variety of devices, including, but not limited to patches, cotton balls, wipes, chambers and the like. An example of an occlusive patch that can be used to expose the skin to a topical skin care product is a Hill Top chamber® (Hill Top Research, Cincinnati, Ohio). This patch includes a molded plastic chamber within which a non-woven Webril® pad (BBA Nonwovens, Simpsonville, SC) holds the topical skin care product. The chamber is applied to the skin using a semi-occlusive, hypo-allergenic adhesive tape. The topical skin care product may be applied to the skin for various lengths of time, for example, ranging from one minute to 24 hours. Examples of exposure to external aggressions include, but are not limited to, scrubbing with wash cloths, wipes, pouffs, bathing implements, exposure of skin to the sun, smoke, wind or other environmental agents, or friction resulting from the wear of clothing. The length of exposure of the external aggression may range, for example, from one minute to one year and in some cases may encompass even a lifetime of exposure.

Following the exposure of the skin to the topical skin care product or external aggression, a non-invasive collection device is used to absorb or collect inflammatory mediators contained in skin secretions present on the skin surface. An example of a suitable non-invasive device for absorbing or collecting mediators form the skin is a mild-adhesive-coated microporous plastic film, such as Sebutape® (available from Cuderm Corporation, Dallas, Texas). The collection of the inflammatory mediator can occur at varying times after exposure to a topical skin care product or external aggression, ranging from immediately after exposure to 24 hours or longer after exposure. For example, the inflammatory mediator may be collected one hour, three hours or 24 hours after exposure of the skin to the topical skin care product or external aggression. We have found in a study of the kinetics of $PGE_2$ expression that the levels of $PGE_2$ were highest 24 hours following exposure versus the shorter times. However, in cases of cumulative effects, it may be appropriate to take measurements as a function of time throughout a lifetime of exposure.

Other suitable non-invasive collection devices include devices selected from the group consisting of an uncoated non-porous plastic film, an uncoated microporous plastic film, an adhesive-coated non-porous plastic film, an adhesive-coated microporous plastic film, a woven fibrous web, a non-woven fibrous web, a natural sponge, a synthetic sponge and a plastic foam.

Following collection by the non-invasive collection device, the level of the inflammatory mediator is analyzed using one or more of a variety of different analytical techniques. Exemplary analytical techniques useful in the practice of the invention include, but are not limited to immunoassay techniques and instrumental analytical techniques.

Exemplary immunoassay techniques useful in the practice of the invention include immunoassay techniques such as radioimmimoassay (RIA), fluorescence immunoassay (FIA), enzyme immunoassay (EIA), and enzyme linked immunoabsorbent assay (ELISA). Immunoassay techniques are used to identify and quantify analytes using antibody-antigen reactions. The antibodies used in the immunoassays may be either polyclonal or monoclonal antibodies. In one approach known as a "sandwich" immunoassay, an antibody to the analyte to be measured is immobilized onto a solid surface such as a bead or a plastic (microtiter) plate. A test sample containing the analyte to be measured is mixed with the antibody beads or placed in the plastic plate, resulting in the formation of an antibody-analyte complex. An indicator reagent composed of a second antibody that carries, or is conjugated to an indicator is then added to the mixture. The indicator may be a radioisotope for RIA, an enzyme for EIA or for ELISA, or a fluorophore for EIA. The most commonly used enzymes in EIA or ELISA are horseradish peroxidase and alkaline phosphatase. The antibody-indicator conjugate binds to the first antibody-analyte complex, free antibody-indicator conjugate is washed away, and the antibody-analyte-antibody-indicator complex is quantified using a method compatible with the indicator reagent. For example, in the case of an enzyme immunoassay, quantitation is effected by the addition of a substrate that reacts with the enzyme.

In an alternate approach known as a competitive binding immunoassay, an analyte in the sample to be measured competes with a known amount of added analyte that has been labeled with an indicator (analyte-indicator conjugate) that binds to the immobilized antibody. After reaction, the free analyte-analyte-indicator solution is washed away from the solid phase. The analyte-indicator on the solid phase or remaining in the wash solution is then used to quantify the amount of analyte present in the sample as measured against a control assay using only an analyte-indicator. This is done using a method appropriate for the assay, for example, enzyme activity, fluorescence, radioactivity, and the like.

In an alternate approach known as the displacement method, a displacement rather than a competitive reaction is used, in which the analyte displaces analyte-indicator bound to the antibody.

Conjugation involves the chemical linkage of the antibody or antigen to another molecule such as a radioisotope; an enzyme such as peroxidase, alkaline phosphatase or glucose oxidase; or a fluorophore such as fluoroscein or rhodamine B. Noncovalent methods for conjugating antibodies and antigens may also be used. For example, in one approach, the antigen and/or the antibody are labeled using either biotin or strepavidin, and the conjugates may then be used in either a competitive or displacement type immunoassay. The advantage in the use of such conjugates is the extremely high affinity constant of the avidin-biotin complex, estimated at approximately $10^{14}$ L/mol.

Traditionally, EIAs have been developed with multi-well plates, for example, 96-well microtiter plates, which provide the immobilization support for the assay, the reaction vessel. and, when linked to a spectrophotometer-based reader, a rapid means for detecting and quantifying the color resulting from interaction of a substrate with the antibody-antigen-enzyme complex.

There are many variations to the basic EIA. For example, an EIA may utilize enzyme amplification to increase the speed and sensitivity of an immunoassay. In this approach, the enzyme label in the EIA produces a substance that triggers a second enzyme-based system that can generate large quantities of color in a very short time. Thus, the product of the enzymatic activity of the antigen-antibody-enzyme complex does not need to be directly detected; rather, it can serve as a catalyst to begin the second reaction. The second enzyme system can be present in relatively large quantities, facilitating rapid color formation, because the second enzyme is silent and noninteractive with the assay until the first reaction product turns it on.

Another approach to enzyme activation, termed prosthetic group label immunoassay (PGLIA), allows the EIA to be carried out in a homogeneous format in which no washing steps are required. The advantage of this approach is that no separation steps are required for the assay.

An exemplary immunoassay that may be used for quantifying levels of $PGE_2$ in the practice of the method of the invention is the High Sensitivity $PGE_2$ Enzyme Immunoassay # 93001 from Assay Designs, Inc. of Ann Arbor, MI.

Exemplary instrumental analytic techniques useful in the practice of the invention include gas chromatography/mass spectrometry (GC/MS), high performance liquid chromatography, (HPLC), thin layer chromatography (TLC) and the like, as well as colorimetric or spectroscopic methods. An exemplary colorimetric method useful in the practice of the invention is the total protein assay using bicinchoninic acid (BCA) (Kit #23225, Pierce Chemical Company, Rockford, IL).

EXAMPLES

The advantages of the invention and specific embodiments of the method and kit of present invention are illustrated by the following examples. It will be understood, however, that the invention is not confined to the specific limitations set forth in the individual examples, but rather, is defined within the scope of the appended claims.

In the examples described below, levels of $PGE_2$, IL-$1\alpha$ and protein were determined using Assay Designs, Inc. Immunoassay kit #93001, Endogen, Inc. Immunoassay kit EH2-IL1A and the Pierce BCA Protein Assay Reagent kit, respectively. A summary of these assays is provided below:

Assay Designs. Inc, $PGE_2$ Enzyme Immunoassay:

The analyses are conducted in the supplied Goat anti-Mouse IgG 96-well microtiter plate.

A series of eight $PGE_2$ standard solutions is prepared by serial dilution of a standard solution containing 50,000 pg/mL $PGE_2$. The eight standard solutions contain 1000, 500, 250, 125, 62.5, 31.25, 15.63, and 7.81 pg/mL $PGE_2$, respectively.

100 $\mu$L of assay buffer are pipetted into the non-specific binding (NSB) and $B_0$ (0 pg/mL) wells.

100 $\mu$L of each of the $PGE_2$ standards is pipetted into the appropriate wells.

100 $\mu$L of each of the samples is pipetted into the appropriate wells.

50 $\mu$L of assay buffer is pipetted into the NSB wells.

50 $\mu$L of blue conjugate (a blue solution of alkaline phosphatase conjugated with $PGE_2$) is pipetted into each well except for the total activity (TA) and blank wells.

50 $\mu$L of yellow antibody (a yellow solution of a monoclonal antibody to $PGE_2$) is pipetted into each well except for the blank, TA and NSB wells.

The plate, covered with the provided plate sealer, is incubated overnight (18 to 24 hours) at 4°C.

The wells are emptied and are washed by adding 200$\mu$L of wash solution to each well. The washes are repeated two more times for a total of three washes. After the final wash, the wells are emptied and the plate is firmly tapped on a lint-free paper towel to remove any remaining wash buffer.

5 $\mu$L of blue conjugate is added to the TA wells.

200 $\mu$L of p-NPP (p-nitrophenyl phosphate) substrate solution is added to each well.

The plate is covered and is incubated at 37°C for one hour without shaking.

50 $\mu$L of stop solution (trisodium phosphate) is added to each well. The plates are read immediately after the stop solution is added.

The plate is blanked in the plate reader against the blank wells. The optical density of the wells is read at 405 nm, preferably with correction between 570 and 590 nm.

The calculation of the concentration of $PGE_2$ in the samples is preferably conducted with by an immunoassay software package utilizing a 4-parameter logistic curve fitting program such as "AssayZap" sold by Biosoft (Ferguson, MO). Alternatively, the concentration of $PGE_2$ may be calculated as follows:

The average net optical density (OD) bound for each standard and sample is calculated by subtracting the average NSB OD from the average OD bound.

$$Average\ Net\ OD = Average\ Bound\ OD - Average\ NSB\ OD$$

The binding of each pair of standard wells as a percentage of the maximum binding wells ($B_0$) is calculated using the following formula:

$$Percent\ Bound = \frac{Net\ OD}{Net\ B_0\ OD} \times 100$$

The percent bound vs. concentration of $PGE_2$ for the standards is plotted using logit-log paper. The concentration of $PGE_2$ in the unknowns may be determined by interpolation.

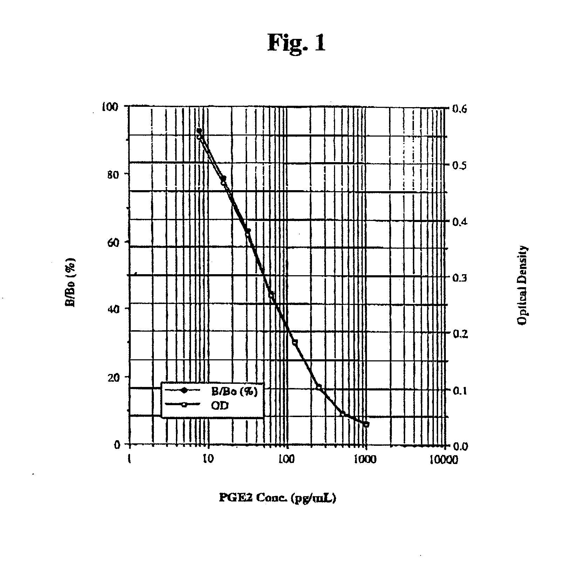

A typical standard curve of percent bound vs. $PGE_2$ concentration is shown in Fig. 1.

Endogen, Inc, Interleukin-1$\alpha$ ELISA

The assay is performed in the provided anti-human IL-1$\alpha$ pre-coated stripwell plate.

For the preparation of the calibration curve, standards are prepared by serial dilution having IL-1$\alpha$ concentrations of 400, 160, 64, 25.6, 10.24, 6.12, 3.06 and 0 pg/mL.

50 $\mu$L of each standard and sample are added in duplicate to test wells. The plate is covered with an adhesive plate cover and is incubated for one hour at room temperature, 20 to 25°C.

50 $\mu$L of biotinylated antibody reagent is added to each of the wells. The plate is covered with an adhesive plate cover and is again incubated for one hour at room temperature.

At the end of the incubation period, the plate cover is carefully removed and the plate is washed three times with wash buffer. After the third wash is removed, the plates are patted onto paper towels or other absorbent material.

100 $\mu$L of streptavidin-horseradish peroxidase (HRP) is added to each of the wells. A new adhesive plate cover is attached, and the plate is incubated for 30 minutes at room temperature.

At the end of the incubation period, the plate cover is removed and the wells are washed three times with wash buffer. The plates are then patted onto paper towels or other absorbent material.

100 $\mu$L of 3,3',5,5'-tetramethyl benzidine dihydrochloride (TMB) substrate solution is added to each well. The plate is allowed to react with the substrate for 30 minutes in the dark at room temperature. After 30 minutes, the reaction is stopped by the addition of 100 $\mu$L of stop solution to each well.

Within thirty minutes of stopping the reaction; the plate is read on a plate reader set at 450 and 550 nm. The reading at 550 nm is subtracted from the reading at 450 nm. Reading at the dual wavelengths corrects for optical imperfections in the microtiter plate.

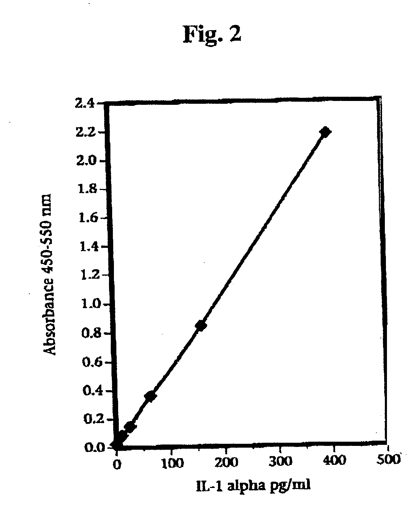

The standard curve is used to determine the amount of IL-1$\alpha$ in an unknown sample. The standard curve is determined by plotting the average absorbance (450-550 nm) obtained for each of the standards vs. the IL-1$\alpha$ concentration. A typical plot is shown in Fig. 2. An unknown is determined manually using graph paper or with a curve-fitting statistical software package to plot a four-parameter logistic curve. The amount of IL-1$\alpha$ in each sample is determined by interpolating from the absorbance value to the IL-1$\alpha$ concentration using the standard curve.

Protein Assay with Pierce BCA Reagent

The Pierce BCA protein assay combines the well-known reduction of $Cu^{+2}$ to $Cu^{+1}$ by protein in an alkaline medium with the highly sensitive and selective colorimetric detection of the cuprous ion using a reagent containing bicinchoninic acid. The purple-colored reaction product of this assay is formed by the chelation of two molecules of BCA with one cuprous ion. The water-soluble complex exhibits a strong absorbance at 562 nm that is linear over a broad working range of protein concentrations.

The BCA protein assay consists of two BCA reagents and an albumin standard. BCA reagent A contains sodium carbonate, sodium bicarbonate, bicinchoninic acid and sodium tartarate in sodium hydroxide solution. BCA reagent B contains 4% cupric sulfate. The BCA working reagent (WR) is prepared by mixing 50 parts of BCA Reagent A with one part of BCA Reagent B. The WR is stable for several days in a closed container at room temperature.

The albumin standard contains Bovine Serum Albumin (BSA) at a concentration of 2.0 mg/mL (2,000 $\mu$g/mL) in 0.9% saline and 0.05% sodium azide. The standard may be diluted with diluent to prepare standards down to a concentration of 5 $\mu$g/mL.

25 μL of each standard and unknown are pipetted into the appropriate microwell plate wells. 25 μL of diluent is used for the blank wells.

200 μL, of WR is added to each well. The plate is mixed well with a plate shaker for 30 seconds. The plate is covered and incubated at 37°C for 30 minutes. The plate is then cooled to room temperature and the absorbance of each well is read on a plate reader at 562 nm.

The average 562 nm absorbance of the blank is subtracted from the reading of all other individual standards and unknown samples. A standard curve is prepared by plotting the average blank-corrected reading for each of the standards vs. its concentration in μg/mL. The standard curve is used to determine the protein concentration of each of the unknown samples. Alternatively, curve-fitting software referred to hereinabove may be used for unknown quantitation.

EXAMPLE 1 - Method of Measuring the Irritation Effect of Water on the Skin

As a means to determine the sensitivity of the method to measure sub-clinical effects, two mild fluids were chosen to determine their inflammatory potential after product exposure. Some of the mildest fluids that could be envisioned would be different types of water, present during skin cleansing, as well as a major component in many topical skin care products. In this example, we measured the inflammatory response of the skin using $PGE_2$ as a marker after exposure of the skin to deionized water and tap water.

The following method was followed;

1. Baseline (corresponding to an untreated control) levels of $PGE_2$ contained in secretions present on the skin surface were measured along the lower volar forearms of test panelists by placing a mild adhesive-coated microporous plastic film, Sebutape® (Cuderm Corporation, Dallas, Texas), on the skin with the adhesive side down, making direct contact with the skin. The Sebutape® was removed from the skin after 1 minute and then placed in a vial containing 500 μL saline. This Sebutape® collection process was repeated three more times with a 1-minute contact time for each Sebutape®. The collected samples were stored at -70 °C until analysis (described below).

2. Hill Top chambers® (Hill Top Research, Cincinnati, Ohio) containing 400 μL of the test fluid (deionized or tap water) were applied to the lower volar forearms of the test panelists. After 4 hours of exposure, the Hill Top chambers® were removed and the test sites were rinsed with tap water and patted dry.

3. Twenty four hours after the Hill Top chambers® were removed and the arm was rinsed and patted dry, skin secretions containing $PGE_2$ expressed on the skin surface were collected using the Sebutape absorption method described previously in Step #1 above. While the inflammatory mediators may be collected at any time after exposure of the test product or process to the skin, it is preferable to measure the levels of $PGE_2$ expressed on the skin approximately 24 hours after exposure, as a study of the kinetics of $PGE_2$ expression revealed that the levels of $PGE_2$ were highest 24 hours after exposure versus after shorter times.

The collected Sebutape® samples were analyzed using the following methods. After thawing, the samples were sonicated for 15 minutes and vortexed vigorously. The solutions, containing extracted proteins and eicosanoids, were analyzed for levels of $PGE_2$ via immunoassay (Assay Designs High Sensitivity $PGE_2$ Enzyme Immunoassay # 93001) and levels of total protein using the bicinchoninic acid (BCA) protein assay (Kit #232257 Pierce Chemical Company, Rockford, IL).

Four Sebutape® samples were taken from each test site. The measured levels of $PGE_2$ (in pg/mL) were normalized against the total amount of protein (in μg/mL) extracted from each Sebutape® strip. The four normalized values were then averaged to determine the levels of $PGE_2$ (in pg/μg) expressed for each test site. To account for person-to-person variability, $PGE_2$ levels for each panelist were further normalized relative to the levels of $PGE_2$ measured for the untreated control.

The level of irritation elicited by tap water compared to that of deionized water using the method described above is shown in Table 1.

Table 1 -

| Normalized levels of $PGE_2$ (dimensionless units) | | |
|---|---|---|
| Panelist | Deionized Water | Tap Water |
| 1 | 0.40 | 0.52 |
| 2 | 0.56 | 0.85 |

Table 1 - (continued)

| Normalized levels of PGE$_2$ (dimensionless units) | | |
|---|---|---|
| Panelist | Deionized Water | Tap Water |
| 3 | 0.62 | 0.89 |
| 4 | 0.69 | 0.72 |
| 5 | 0.92 | 1.41 |
| 6 | 0.84 | 1.51 |
| 7 | 0.93 | 1.40 |
| 8 | 1.71 | 1.45 |
| 9 | 1.01 | 2.33 |
| 10 | 0.94 | 2.08 |
| Average | 0.86 | 1.32 |

As shown in Table 1, the method is able to differentiate between the levels of inflammation or irritation elicited by different types of water tap water causes approximately 35% more of the inflammatory mediator PGE$_2$ to be expressed on the skin surface than deionized water.

EXAMPLE 2 - Possible Interactions of Anionic Surfactants with Interleukin-1 alpha (IL-1$\alpha$)

One of the concerns of using IL-1$\alpha$ as a marker of inflammation or irritation is its potential interaction with surfactants often contained in topical skin care compositions. This example illustrates this interaction as measured by the apparent levels of IL-1$\alpha$ expressed on the skin as a function of the concentration of sodium lauryl sulfate (SLS, Stepan Co., Northfield, IL) in a test fluid.

The level of irritation caused by dilute solutions of SLS as a function of SLS concentration was assessed using the method described in Example 1, with the exception that the level of Interleukin-1$\alpha$ (IL-1$\alpha$) was used to assess the degree of irritation, rather than PGE$_2$. The levels of IL-1$\alpha$ were measured via immunoassay (Human IL-1$\alpha$ Kit #EH2-IL1a, Endogen, Inc., Woburn, MA). As with PGE$_2$, the measured levels of IL-1$\alpha$ (in pg/mL) were normalized against the total amount of protein (in $\mu$g/mL) extracted from each Sebutape® strip. To account for person-to-person variability, IL-1$\alpha$ levels for each treatment site were further normalized using the baseline levels of IL-1$\alpha$ measured for the untreated control. The data are shown below in Table 2.

Table 2 -

| Normalized levels of IL-1alpha (dimensionless units) | |
|---|---|
| Concentration of SLS in Deionized Water (w/w %) | Levels of IL-1$\alpha$ (dimension less units) |
| 0 | 1.35 |
| 5 | 2.83 |
| 10 | 2.71 |
| 20 | 1.00 |

Table 2 shows the dose dependent data of IL-1$\alpha$ after exposure of the skin to various concentrations of SLS. The amount of the inflammatory marker or response is expected to increase as the level of the anionic surfactant, SLS, increases. While this is observed at low SLS levels, this is not seen at the highest concentration of SLS tested in the study, raising a potential issue as to the robustness of the method. While wishing not to be bound by theory, it is thought that high concentrations of anionic surfactants, such as SLS, may interact with proteins, such as IL-1$\alpha$, causing denaturation of the proteins. This process would affect the immunoassays, and could result in the lower apparent levels of IL-1$\alpha$ that are observed in this experiment at the highest SLS concentration. When analyzing for levels of IL-1$\alpha$ using immunoassays, the SLS may change the conformation of the protein, rendering it unrecognizable by the antibody used in the assay. Thus, using the levels of IL-1$\alpha$ to assess the degree of irritation is limited due to these potential surfactant-protein interactions.

EXAMPLE 3 - Inflammation due to a Mild Facial Cleanser

The method of Example 1 was extended to measure the level of inflammation or irritation elicited by a dilute solution (8% w/w in deionized water) of a mild facial cleanser (Neutrogena Fresh Foaming Cleanser, Neutrogena Corporation, Los Angeles, California) compared to that of deionized water.

The ingredients of the cleanser composition are listed in Table 3.

Table 3 -
Composition of the mild facial cleanser

Purified Water
Glycerin
Lauryl Glucoside
Decyl Glucoside
Cocomidopropyl Betaine
Cocamide DEA
Glycereth-7
Ammonium Laureth Sulfate
Sodium Cocoyl Sarcosinate
Glycol Stearate
PEG-120 Methyl Glucose Dioleate
Tetrasodium EDTA
DMDM Hydantoin
Citric Acid
Fragrance

The level of irritation elicited by the facial cleanser compared to that of deionized water as indicated by expressed levels of $PGE_2$ using the methods described above is shown in Table 4.

Table 4 -

| Normalized levels of PGE2 (dimensionless units) | | |
|---|---|---|
| Panelist | Deionized Water | Facial Cleanser |
| 1 | 1.98 | 1.81 |
| 2 | 1.48 | 1.26 |
| 3 | 1.30 | 0.67 |
| 4 | 0.83 | 2.11 |
| 5 | 1.49 | 1.72 |
| 6 | 2.12 | 3.07 |
| 7 | 1.25 | 2.38 |
| 8 | 1.60 | 3.03 |
| 9 | 1.77 | 3.05 |
| 10 | 1.45 | 0.85 |
| Average | 1.53 | 2.00 |

The results in Table 4 show that the inflammation response of these mild products can be differentiated using the aforementioned methodology. Exposure of the skin to this mild facial cleanser results in greater inflammation or irritation than deionized water; however, the level of irritation caused by the mild cleanser is only about 24% greater than that caused by deionized water alone.

It is important to realize that the numerical irritation results cited here are not absolute; rather, they were obtained for a particular population of panelists at a particular time of year. It is known that different people will exhibit different inflammatory responses upon exposure to the same topical skin care product. In addition, it is also known that insults to the stratum corneum during warmer, more humid times of the year (*i.e.,* summer) will have a lesser effect than during colder, dryer times of the year (*i.e.,* winter). Thus, it is important not to cross-compare the actual values measured for each of the different studies unless these variables are taken into account.

EXAMPLE 4 - Exposure to External Aggressions

The method of the invention may also be extended to external aggressions, for instance, to test the irritation provided by an applicator for a topical skin care product. In this example, the level of inflammation or irritation caused by scrubbing with a baby washcloth (Gerber® Baby Washcloth) was compared to the level of irritation caused by using a regular washcloth (St.Mary's® - Division of Fieldcrest Cannon, Inc.).

The methods used to assess inflammation or irritation were similar to the method of Example 1, with the exception that instead of using a Hill Top Chamber® to apply the irritant to the arm, the following scrubbing protocol was used- Each washcloth (Baby Cotton Washcloth vs. Regular Cotton Washcloth) was saturated with an 8% (w/w) dilution of Johnson's® Head-to-Toe™ Baby Wash (Johnson & Johnson Consumer Products Company, Sidllman, New Jersey, ingredients list shown in Table 5) in tap water. Each site on the volar forearm was scrubbed for 2 minutes. After washing; the arm was rinsed with water and patted dry. $PGE_2$ levels were analyzed and are reported in Table 6.

Table 5 -
Composition of the baby wash

Water
PEG-80 Sorbitan Laurate
Sodium Laureth Sulfate
Cocamidopropyl Betaine
PEG-150 Distearate
Sodium Lauroampho PG-Acetate Phosphate
Glycerine
Polyquaternium-10
Fragrance
Quaternium-15
Tetrasodium EDTA

Table 6 -

| Normalized levels of PGE2 (dimensionless units) | | |
|---|---|---|
| Panelist | Baby Washcloth | Regular washcloth |
| 1 | 0.84 | 1.31 |
| 2 | 1.08 | 0.88 |
| 3 | 1.13 | 1.19 |
| 4 | 1.02 | 1.08 |
| 5 | 0.87 | 1.29 |
| 6 | 1.35 | 0.89 |
| 7 | 1.17 | 0.92 |
| 8 | 0.70 | 1.13 |
| 9 | 1.17 | 1.29 |
| 10 | 0.57 | 1.10 |
| Average | 0.99 | 1.11 |

As seen in Table 6, the method of the invention is useful to distinguish between levels of inflammation or irritation caused by exposure to an external aggression. The method of the invention indicates a directional difference between scrubbing with the baby washcloth versus the regular washcloth; scrubbing with a baby washcloth causing less irritation than scrubbing with the regular washcloth.

EXAMPLE 5 - External Aggressions in Conjunction with Topical Skin Care Products

The method of the invention may be applied to a comparison of different topical skin care products with the concomitant exposure to an external aggression. In this example, scrubbing with a dilute solution of a baby bath was compared to scrubbing with tap water alone. The baby bath was a 10% (w/w) dilution of Johnson's® Head-to-Toes™ Baby Wash (Johnson & Johnson Consumer Products Company, Skillman, New Jersey, ingredient list shown previously in Table 5) in tap water.

Inflammation or irritation was assessed as described in Example 1, with the exception that rather than using a Hill Top Chamber®, 2 mL of water or 2 mL of a dilute solution of a baby bath were used to wash the volar forearm using a Dia-stron® Wash Simulator (cyberDERM Inc., Media, Pennsylvania). Each site was scrubbed for 2 minutes. After scrubbing, the arm was rinsed with water and patted dry. $PGE_2$ results are listed in Table 7.

Table 7 -

| Normalized Levels of $PGE_2$ (Dimensionless units) | | |
|---|---|---|
| Panelist | Scrubbing with Tap Water | Scrubbing with Baby Bath |
| 1 | 0.68 | 0.62 |
| 2 | 0.76 | 0.62 |
| 3 | 1.22 | 0.50 |
| 4 | 1.28 | 0.30 |
| 5 | 1.29 | 0.67 |
| 6 | 0.90 | 0.65 |
| 7 | 0.47 | 0.44 |
| 8 | 1.40 | 0.66 |
| 9 | 0.21 | 0.11 |
| 10 | 0.11 | 0.17 |
| Average | 0.83 | 0.47 |

As demonstrated in Table 7, the method of the invention may be used to test the relative irritation of topical skin care products during concomitant exposure to an external aggression. Washing with tap water alone is shown to cause more irritation than washing with the mild baby bath.

EXAMPLE 6 - Evaluation of External Aggressions via Levels of IL-1$\alpha$

Previous examples have demonstrated the ability of $PGE_2$ to distinguish between the levels of inflammation or irritation due to exposure to a topical skin care product or exposure to an external aggression. In addition, previous work has shown a correlation between cytokine levels, for example IL-1$\alpha$, and the amount of inflammation caused by sodium lauryl sulfate application, as well as the use of cytokines to characterize severe disease conditions. In this example, we show how the method of the invention, may be applied to monitoring the levels of IL-1$\alpha$ as a measure of the level of inflammation or irritation caused by external aggressions, for instance the method of applying the topical skin care product to the skin. In this example, the level of inflammation or irritation caused by scrubbing with a baby washcloth is compared to the level of irritation when using a regular washcloth.

The relative irritation caused by the two applicators was assessed using the method of Example 1 with the exception that the following scrubbing protocol was used in place of the Hill Top Chamber®. Each washcloth (Baby Cotton Washcloth - Gerber® Baby Washcloth, Regular Cotton Washcloth - St-Mary's®- Division of Fieldcrest Cannon, Inc.) was saturated with an 8% (w/w) dilution of Johnson's® Head-to-Toe™ Baby Wash (Johnson & Johnson Consumer Products Company, Skillman, New Jersey, ingredients listed previously in Table 5) in tap water. Each site on the volar forearm was scrubbed for 2 minutes. After washing, the arm was rinsed with water and patted dry. Also contrary to Example 1, IL-1$\alpha$ levels were monitored as a measure of the level of inflammation or irritation, rather than PGE2. The levels of IL-1$\alpha$ were measured via immunoassay (Endogen Human IL-1alpha Kit #EH2-IL1a), and these values are reported in Table 8.

Table 8 -

| Normalized Levels of IL-1$\alpha$ (dimensionless units) | | |
|---|---|---|
| Panelist | Baby Washcloth | Regular Washcloth |
| 1 | 0.73 | 0.52 |
| 2 | 1.59 | 0.79 |
| 3 | 0.97 | 1.44 |
| 4 | 0.92 | 1.78 |
| 5 | 0.50 | 0.46 |
| 6 | 0.42 | 0.36 |
| 7 | 0.27 | 1.00 |
| 8 | 1.54 | 1.96 |
| 9 | 1.23 | 2.62 |
| Average | 0.91 | 1.22 |

As seen in Table 8, measurements of IL-1$\alpha$ can be used to distinguish between levels of inflammation or irritation caused by exposure to an external aggression such as scrubbing with different types of cloths. Scrubbing with a baby washcloth causes less irritation than scrubbing with a regular washcloth.

In addition, a more complete indication of inflammation or irritation caused by exposure to a topical skin care product and/or to an external aggression can be obtained by monitoring the levels of IL-1$\alpha$ in conjunction with PGE$_2$. Combining the results shown previously for PGE$_2$ in Example 4 with this example provides a more comprehensive understanding of the effects of scrubbing with different types of cloth on inflammation, since we are measuring primary pro-inflammatory mediators of the cytokine pathway as well as the arachidonic acid pathway.

**Claims**

1.  A method for measuring a marker of clinical or sub-clinical inflammation or irritation of mammalian skin, comprising the steps of:

    (a)  collecting secretions from the surface of the skin using a non-invasive collection procedure, said non-invasive collection procedure utilizing a non-invasive collection device; and
    (b)  analyzing the level of at least one eicosanoid in the secretions collected from the skin surface by said device.

2.  The method of claim 1, wherein said non-invasive collection device is a device selected from the group consisting of uncoated non-porous plastic film, an uncoated microporous plastic film, an adhesive-coated nonporous plastic film, an adhesive-coated microporous plastic film, a woven fibrous web, a non-woven fibrous web, a natural sponge, a synthetic sponge and a plastic foam.

3.  The method of claim 1 or claim 2, wherein said eicosanoid is prostaglandin.

4.  The method of any one of claims 1 to 3, wherein the level of eicosanoid is analyzed using at least one of RIA, EIA and ELISA.

5.  The method of any one of claims 1 to 3, wherein the level of eicosanoid is analyzed using at least one of GC/MS, HPLC, and TLC.

6.  The method of any one of claims 1 to 5, further comprising the step of analyzing the level of at least one cytokine in the secretions collected from the surface of said skin by said device.

7.  A method of claim 6, wherein said cytokine is interleukin-1$\alpha$ and said eicosanoid is prostaglandin E$_2$.

8. A method for measuring sub-clinical or clinical inflammation or irritation of mammalian skin from exposure of said skin to a topical skin care product, exposure to an external aggression or combinations thereof, said method comprising the steps of:

    (a)    collecting secretions from the surface of said skin using a non-invasive collection procedure comprising a non-invasive collection device;
    (b)    measuring a baseline level of eicosanoid in the secretions collected from the surface of said skin;
    (c)    exposing said skin to a topical skin care product, to an external aggression or combinations thereof;
    (d)    collecting secretions from the surface of said skin using a non-invasive collection device after step (c);
    (e)    measuring the level of eicosanoid in the secretions collected from the surface of said skin after step (c); and
    (f)    comparing the level of eicosanoid determined in step (e) with the level of eicosanoid determined in step (b).

9. A kit for measuring a marker of sub-clinical or clinical inflammation or irritation of mammalian skin, said kit comprising:

    (a)    a non-invasive collection device for collecting secretions from the surface of said skin; and
    (b)    an immunoassay for measuring levels of eicosanoid in said secretions.

10. The kit of claim 9 wherein said kit is used to measure the sub-clinical or clinical inflammation or irritation of mammalian skin due to exposure of said skin to at least one topical skin care product, exposure to at least one external aggression or combinations thereof.

# Fig. 1

PGE2 Conc. (pg/mL)

Fig. 2